# EUROPEAN PATENT SPECIFICATION

(11) **EP 2 297 074 B1**
(45) Date of publication and mention of the grant of the patent: **22.10.2014**
(21) Application number: 09753983.7
(22) Date of filing: 02.06.2009
(51) Int. Cl.: C07C 29/56, C07C 33/048

(54) **PROCESS FOR THE REARRANGEMENT OF ALLYL ALCOHOLS**
VERFAHREN ZUR NEUANORDNUNG VON ALLYLALKOHOLEN
PROCÉDÉ DE RÉORGANISATION D'ALCOOLS D'ALLYLE

(30) Priority: 30.05.2008 EP 08009916
(43) Date of publication of application: 23.03.2011
(73) Proprietor: DSM IP Assets B.V., 6411 TE Heerlen (NL)
(72) Inventor: AQUINO, Fabrice, F-68950 Reiningue (FR); BONRATH, Werner, Freiburg 79115 (DE)
(74) Representative: Steck, Melanie
(86) International application number: PCT/EP2009/056730
(87) International publication number: WO 2009/144328

(56) References cited:
- US-A- 2 567 572
- DATABASE CA [Online] CHEMICAL ABSTRACTS SERVICE, COLUMBUS, OHIO, US; SKODA, ALOJZ ET AL: "Allylic rearrangement of 3-methyl-1-penten-4-yn-3-ol to cis-3-methyl-2-penten-4-yn-3-ol catalyzed by a cation-exchange resin" XP002505284 retrieved from STN Database accession no. 1991:228365 cited in the application & CS 269 025 B1 (CZECH.) 11 April 1990 (1990-04-11)
- DATABASE CHEMABS CHEMICAL ABSTRACTS SERVICE, COLUMBUS, OHIO, US; XP002505285 Database accession no. 1960:86241 & SU 126 227 A 10 February 1960 (1960-02-10)
- M. HART: "Sulfonated poly(styrene-co-divinylbenzene)ion-exchang e resins:acidities and catalytic activities in aqueous reactions", JOURNAL OF MOLECULAR CATALYSIS A:CHEMICAL, vol. 182-183, 2002, pages 439-445,

## Description

The present invention relates to a process for isomerizing a pent-1-en-3-ol to a mixture of the isomers Z-pent-2-en-1-ol and E-pent-2-en-1-ol.

Allyl alcohols, especially tertiary allyl alcohols, are important intermediates in industrial organic chemistry. It is known that allyl alcohols isomerize in presence of an acid catalysis. This isomerization reaction corresponds to a 1,3 migration of the hydroxyl group and a corresponding shift of the double bond. The migration of a double bond and of a substituent is known for allyl compounds and is generally referred to as an allylic rearrangement. Allylic rearrangements of allyl alcohols are equilibrium reactions.

From an industrial viewpoint 3-methylpent-1-en-4-yn-3-ol is important and of great interest because its allylic rearrangement results in about 85% of Z-3-methylpent-2-en-4-yn-1-ol and about 15% of E-3-methylpent-2-en-4-yn-1-ol. The Z-isomer is a useful intermediate, e.g. for the manufacture of Vitamin A, and the E-isomer is also a useful intermediate, e.g. for the manufacture of astaxanthin, zeaxanthin and other carotenoids. The isomers may be separated from each other by physical means, e.g. by fractional distillation.

EP-A-0 860 415 generally describes the isomerization of allyl alcohols to the Z- and E-isomers. The isomerization reaction is performed in an aqueous solution in the presence of protonic acids at a pH in the range of from 2 to 5. The process is predominantly used to convert primary or secondary allyl alcohols to tertiary allyl alcohols. It is further described that a solvent may be present, e.g. in case an allyl alcohol of low solubility in water is used. However, the solvent must be miscible with water in the mixing ratio employed. This method does not seem appropriate to isomerize 3-methylpent-1-en-4-yn-3-ol as this is a tertiary allyl alcohol.

Czech patent application publication no. 269 025 is concerned with a method for producing Z-3-methylpent-2-en-4-yn-1-ol by means of the acid catalyzed allylic rearrangement of 3-methylpent-1-en-4-yn-3-ol. The isomerization reaction is conducted in an organic solvent in the presence of a strongly acidic cation exchanger swollen by water. The organic solvent employed is an aromatic hydrocarbon, e.g. benzene, or a halogenated aliphatic hydrocarbon. However, the reaction is performed at incomplete conversion of no more than 70% and the maximum yield of 3-methylpent-2-en-4-yn-1-ol reported in the examples is only about 52%.

In Chem. Papers 43 (6) 743-751 (1989) A. Skoda et al. report on a kinetic study of the allyl rearrangement of 3-methylpent-1-en-4-yn-3-ol catalyzed by sodium hydrogensulfate in aqueous solution. This publication only describes scientific experiments and does not give any hint on a possible realization of a corresponding technical process for large scale production. Moreover, the separation of the catalyst for further reuse is difficult.

According to US 2,567,572 the acetylene carbinol of compound XII is rearranged by treating it in an aqueous solution of a mineral acid such as sulfuric acid to form the enyne carbinol compound XIIa as follows:

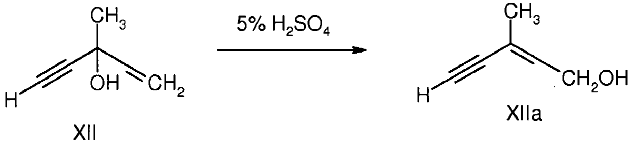

SU 126 227 describes a method for the isomerization of 3-methyl-3-ol-4-yn-penta-ene-1 to 3-methyl-penta-en-2-yn-4-ol-1 using ion-exchange resins, wherein, with the object of acceleration, the process is carried out using polystyrene sulfo ion-exchange resins in an aqueous solution or in a mixture of water and another polar solvent.

It is the object of the present invention to provide an alternative process for isomerizing a pent-1-en-3-ol, especially 3-methylpent-1-en-4-yn-3-ol, to a mixture of Z-pent-2-en-1-ol and E-pent-2-en-1-ol, which process provides the isomers in good yields at high conversion and/or with high selectivity. An additional object of the present invention is to establish a catalyst which allows the reducing of waste formation, especially in continuous processing.

The object is met by a process for isomerizing a pent-1-en-3-ol according to formula (1) 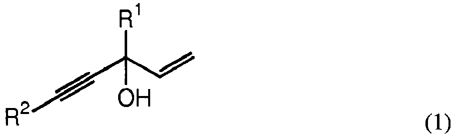 to a mixture of the isomers Z-pent-2-en-1-ol (2) and E-pent-2-en-1-ol (3) according to formulae (2) and (3) 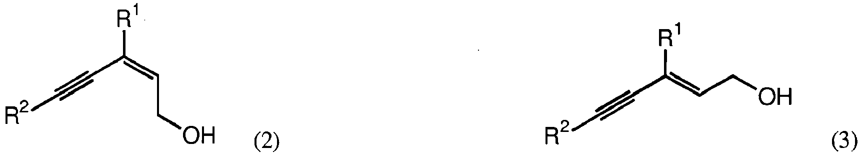 wherein
R¹ is selected from alkyl, aryl, and alkylaryl radicals,
R² is selected from H, alkyl, and aryl radicals;
which process comprises reacting the pent-1-en-3-ol (1) in a solvent and in the presence of a heterogeneous acid catalyst, i.e. that the catalyst is an immobilized solid phase catalyst.

The solvent is a multiphase system, wherein said multiphase system comprises an aqueous phase and an organic solvent phase which form 2 phases. Such a two-phase liquid solvent system is preferred. In the latter case the reaction mixture is a three-phase system: aqueous phase, organic solvent phase and catalyst.

In an especially advantageous embodiment of the present invention the water content in the reaction mixture is at least 5 weight-%.

The organic solvent phase of said multiphase system comprises a water-immiscible organic solvent, whereby the water-immiscible organic solvent is diisopropyl ether.

Usually when using a multiphase (solvent) system problems with mass transport limitation occur. Surprisingly it was recognized that such problems do not occur in the multiphase solvent systems according to the present invention.

Further advantages of the process according to the present invention are the long life time of the catalyst and that the process may also be performed in a continuous way. In contrast to processes where homogeneous acid catalysts are used, a neutralization of the acid is not mandatory at the end of the reaction when heterogenous acid catalysts are used. The neutralization of an acid results in the formation of a salt which has to be removed and disposed.

The present invention is also directed to a process of preparing an isoprenoid selected from vitamin A, vitamin A derivatives, and carotenoids, which process comprises isomerizing a pent-1-en-3-ol as described above, isolating the Z-pent-2-en-1-ol and E-pent-2-en-1-ol, and converting the Z-pent-2-en-1-ol or E-pent-2-en-1-ol to the corresponding isoprenoid.

The present invention further relates to a process of preparing an isoprenoid selected from vitamin A, vitamin A derivatives, and carotenoids, which process comprises isomerizing 3-methylpent-1-en-4-yn-3-ol as described above, isolating the Z-3-methylpent-2-en-4-yn-1-ol and E-3-methylpent-2-en-4-yn-1-ol, and converting the Z-3-methylpent-2-en-4-yn-1-ol or E-3-methylpent-2-en-4-yn-1-ol to the corresponding isoprenoid.

According to a preferred embodiment, R¹ in formulae (1), (2) and (3) is selected from C₁ to C₁₅ alkyl radicals, preferably C₁ to C₅ alkyl radicals (more preferably methyl radicals), and a phenyl radical; R² is selected from H, C₁ to C₁₅ alkyl radicals, preferably C₁ to C₅ alkyl radicals, and a phenyl radical. The alkyl radicals may be linear, branched or cyclic, preferably they are linear or branched, more preferably they are linear.

Further examples of aryl radicals are naphthyl and tolyl.

An example of an alkylaryl radical is benzyl.

According to the most preferred embodiment, R¹ is a methyl radical and R² is H. In this case, the pent-1-en-3-ol is 3-methylpent-1-en-4-yn-3-ol according to formula (4) 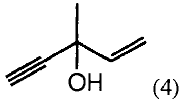 and the corresponding isomers are Z-3-methylpent-2-en-4-yn-1-ol according to formula (5) and E-3-methylpent-2-en-4-yn-1-ol according to formula (6)

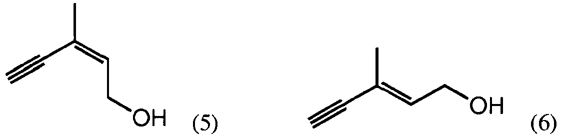

The organic solvent forming the organic solvent phase is the water-immiscible organic solvent diisopropyl ether. The term "water-immiscible" solvent means that the solvent maintains during the isomerization reaction a distinct organic solvent phase in addition to the aqueous phase. Typically, the water-immiscible solvent is miscible in water to an extent of less than 3 g per 100 g of water.

Preferably, the heterogeneous acid catalyst is selected from the group consisting of Brønsted acids on a carrier, strong acidic cation exchanger and polymers having acidic groups, i.e. functionalized polymers. Preferably the catalyst has a pore diameter in the range of from 100 to 500 Ǻ **and/or** a surface area of at least 20 m²/g (measured by nitrogen BET), preferably a surface area in the range of from 20 to 500 m²/g (more preferably in the range of from 25 to 300 m²/g, in the range of from 28 to 300 m²/g, in the range of from 28 to 70 m²/g, in the range of from 30 to 55 m²/g), and/or a total pore volume of at least 0.10 ml/g, preferably a total pore volume in the range of from 0.10 to 0.50 ml/g, more preferably a total pore volume in the range of from 0.15 to 0.45 ml/g, even more preferably a total pore volume in the range of from 0.18 to 0.42 ml/g, most preferably a total pore volume in the range of from 0.20 to 0.40 ml/g.

Even more preferably the acidic group of the catalyst is represented by sulphonic acid groups (-SO₃H) or by any other group having a pKₐ ≤ 4, preferably the acidic function of the catalyst is represented by sulphonic acid groups.

Thus, the catalyst may be an organic sulphonic acid on a carrier or a polymeric resin with functional sulphonic acid groups, whereby the polymeric resins with functional sulphonic acid groups are more preferred. The polymeric resins with functional sulphonic acid groups are also named "polymer bounded sulfonic acid" in the context of the present invention. In a preferred embodiment a styrene-divinylbenzene polymer is used as matrix for the sulphonic acid groups.

A further suitable catalyst is a polymer functionalized with CO₂H groups.

In the context of the present invention the term "functionalized polymer with SO₃H groups" also encompasses polymers, where the SO₃H groups are bound to the polymer backbone via a linker. This linker may be an alkylene (such as e.g. propylene: -(CH₂)₃-), an arylene (such as p-tolylene) or an alkylarylene (such as e.g. benzylene: -CH₂-(C₆H₄)-). The same applies accordingly for the "functionalized polymer with CO₂H groups".

Of all the functionalized polymers cited in the context of the present invention the macroreticular polymers are preferred.

Preferably, the immobilized/solid Brønsted acid for use in the present invention has a pka value ≤ 4, more preferably ≤ 2, even more preferably ≤ 1.5 and most preferably ≤ 1.

Examples of Brønsted acids are organic sulphonic acids, H₂SO₄ and H₃PO₄.

The carrier may be either organic or inorganic. Examples of organic carriers are polymers. Examples of inorganic carriers are oxide carriers such as e.g. SiO₂, Al₂O₃, TiO₂, ZrO₂ and GeO₂.

Examples of Brønsted acids on a carrier are p-toluene sulphonic acid on a polymer (especially on a macroreticular polystyrene copolymer such as a macroreticular polystyrene divinylbenzene copolymer) or propyl sulphonic acid on silica.

Examples of suitable catalysts for the process of the present invention are Amberlyst type polymers, Lewatit, Dowex, und polymer bounded p-TsOH (p-toluene sulphonic acid), as well as Deloxan.

According to the most preferred embodiment, polymer-bounded sulphonic acids, e.g. Amberlyst-type (preferred) and Dowex-type polymers are used as the solid acid catalyst in the present process.

In the most preferred embodiment of the present invention the catalysts Amberlyst 15, 16 and 36, as well as Dowex DR-2030 are used. These are all functionalized styrene divinylbenzene copolymers.

Amberlyst 15 is a bead form, macroreticular, sulphonic acid, strongly acidic ion exchange resin having a concentration of the acid sites (i.e. the sulphonic acid groups) ≥ 4.7 eq/kg, a surface area of 53 m²/g (measured by nitrogen BET), an average pore diameter of 300 Å and a total pore volume of 0.40 ml/g, and commercially available from Rohm & Haas as Amberlyst 15 Dry and Amberlyst 15 Wet. Amberlyst 15 Wet has also a harmonic mean particle size in the range as from 0.600 to 0.850 mm and a uniformity coefficient ≤ 1.70.

Amberlyst 16 is a bead form, macroreticular, sulphonic acid, strongly acidic ion exchange resin having a concentration of the acid sites (i.e. the sulphonic acid groups) ≥ 4.8 eq/kg, a surface area of 30 m²/g (measured by nitrogen BET), an average pore diameter of 250 Å and a total pore volume of 0.20 ml/g, and commercially available from Rohm & Haas as Amberlyst 16 Wet. Amberlyst 16 Wet has also a harmonic mean particle size in the range as from 0.600 to 0.800 mm and a uniformity coefficient ≤ 1.60.

Amberlyst 36 is a bead form, macroreticular, sulphonic acid, strongly acidic ion exchange resin having a concentration of the acid sites (i.e. the sulphonic acid groups) ≥ 5.4 eq/kg, a surface area of 33 m²/g (measured by nitrogen BET), an average pore diameter of 240 Å and a total pore volume of 0.20 ml/g, and commercially available from Rohm & Haas as Amberlyst 36 Dry and Amberlyst 36 Wet. Amberlyst 36 Wet has also a harmonic mean particle size in the range as from 0.600 to 0.850 mm and a uniformity coefficient ≤ 1.60.

Dowex DR-2030 is a bead form, macroreticular, sulphonic acid, strongly acidic ion exchange resin having a concentration of the acid sites (i.e. the sulphonic acid groups) ≥ 4.7 eq/kg, a surface area of 30-31 m²/g (measured by nitrogen BET) and a total pore volume of 0.33-0.35 ml/g, and commercially available from Dow Chemical as Dowex DR-2030 and Dowex Monosphere DR-2030. Dowex Monosphere DR-2030 has also a particle size distribution of 425-525 microns.

Thus, preferred heterogeneous acid catalysts of the present invention are bead form, macroreticular, sulphonic acid, strongly acidic ion exchange resin having 0 a concentration of the acid sites (i.e. the sulphonic acid groups) ≥ 4.0 eq/kg (preferably ≥ 4.5 eq/kg, more even more preferably in the range of from 4.5 to 6.0 eq/kg, most preferably in the range of from 4.5 to 5.5 eq/kg)
and/or 0 a surface area of at least 20 m²/g (measured by nitrogen BET) (preferably in the range of from 20 to 70 m²/g, more preferably in the range of from 28 to 55 m²/g)
and/or 0 a total pore volume of at least 0.10 ml/g (preferably in the range of from 0.10 to 0.50 ml/g, more preferably a total pore volume in the range of from 0.15 to 0.45 ml/g, even more preferably a total pore volume in the range of from 0.18 to 0.42 ml/g, most preferably a total pore volume in the range of from 0.20 to 0.40 ml/g).

Thus, the most preferred heterogeneous acid catalysts of the present invention are bead form, macroreticular, sulphonic acid, strongly acidic ion exchange resin having a concentration of the acid sites (i.e. the sulphonic acid groups) ≥ 4.0 eq/kg (preferably ≥ 4.5 eq/kg, more even more preferably in the range of from 4.5 to 6.0 eq/kg, most preferably in the range of from 4.5 to 5.5 eq/kg), a surface area of at least 20 m²/g (measured by nitrogen BET) (preferably in the range of from 20 to 70 m²/g, more preferably in the range of from 28 to 55 m²/g), an average pore diameter of at least 200 Å (preferably in the range of from 200 to 350 Å) and a total pore volume of at least 0.10 ml/g (preferably in the range of from 0.10 to 0.50 ml/g, more preferably a total pore volume in the range of from 0.15 to 0.45 ml/g, even more preferably a total pore volume in the range of from 0.18 to 0.42 ml/g, most preferably a total pore volume in the range of from 0.20 to 0.40 ml/g).

Furthermore polymers with SO₃H groups as prepared according to the processes of EP-A 507 490 and US 5,081,160, whose contents are incorporated herein by reference, may be also used as catalyst in the isomerization reaction according to the present invention.

Although it is not mandatory, a stabilizer, such as hydroquinone or 2,6-di-tert-butyl-p-cresol or a mixture thereof, may be added to the reaction mixture. If used, the stabilizer, e.g. hydroquinone or 2,6-di-tert-butyl-p-cresol or a mixture thereof, is typically added in an amount of from 1 to 10,000 ppm by weight, preferably from 5 to 1,000 ppm by weight, more preferably from 7 to 500 ppm by weight, and most preferably from 10 to 100 ppm by weight, each based on the total weight of the multiphase system.

Further stabilizers may be other anti-oxidants.

Typically, the solid acid catalyst is mixed with water and added to the process.

Compared to the prior art methods the separation and recycling of the catalyst is much more convenient in the present process. If a multiphase system is used, the solid catalyst and the aqueous phase of the multiphase system may easily be separated from the product layer and recycled. That means that in both variants the catalyst may be recycled. It has been found that the catalyst gives still high yield and conversion even if used several times.

Usually, the pent-1-en-3-ol is soluble in the diisopropyl ether and thus, it is added to the process as a solution in the organic solvent. In one embodiment, the concentration of the pent-1-en-3-ol in the organic solvent ranges from 5 to 80% by weight, preferably from 15 to 60% by weight, more preferably from 20 to 50% by weight, and most preferably from 25 to 45% by weight.

The pent-1-en-3-ol may, however, be also added in the reaction chamber/reactor as such. Preferably the pent-1-en-3-ol is added as a solution in diisopropyl ether.

The various components may be added in any order. For example, the aqueous mixture of the solid acid catalyst may be loaded into the reaction vessel and then the solution of the pent-1-en-3-ol in diisopropyl ether may be added or vice versa. It is also possible to divide the total amount of a component to be added and add the partial amounts at various time points in the process.

If the reaction is carried out discontinuously the mixing of the reaction components is done by agitation of the reaction mixture, e.g. by stirring. Preferably, the isomerization reaction is conducted in a stirred tank reactor.

If the reaction is carried out in continuous manner a plug-flow reactor with a fixed-bed is used, or a continuous stirred tank reactor. In an embodiment of the invention a cascade reactor system is used.

The isomerization reaction is typically conducted at a temperature in the range of from 20 to 80°C, depending on the type of the reaction components used and the other reaction conditions applied. At atmospheric pressure the reaction temperature preferably ranges from 30 to 70°C, more preferably from 35 to 65°C, and most preferably from 40 to 60°C.

The isomerization reaction has depending of the used set-up and conditions reaction times from 1 to 25 h. At a typical temperature range the reaction time is preferably from 1.5 to 8 h, more preferably from 2 to 6 h, and most preferably from 2 to 5 h.

Typically, the process according of the present invention is conducted at a pent-1-en-3-ol conversion of at least 70%, preferably at least 81%, more preferably at least 86%, even more preferably at least 89% and most preferably at least 92%.

In a preferred embodiment of the present invention 3-methylpent-1-en-4-yn-3-ol is isomerized to a mixture of Z-3-methylpent-2-en-4-yn-1-ol and E-3-methylpent-2-en-4-yn-1-ol using the reaction components, amounts and reaction conditions described above, including all the preferred embodiments.

In a more preferred embodiment of the present invention 3-methylpent-1-en-4-yn-3-ol is isomerized to a mixture of Z-3-methylpent-2-en-4-yn-1-ol and E-3-methylpent-2-en-4-yn-1-ol employing diisopropylether as its/their solvent and using the other reaction components, amounts and reaction conditions described above, including all the preferred embodiments.

In a further preferred embodiment of the present invention 3-methylpent-1-en-4-yn-3-ol is isomerized to a mixture of Z-3-methylpent-2-en-4-yn-1-ol and E-3-methylpent-2-en-4-yn-1-ol employing diisopropylether as its/their solvent, Brønsted acids on a carrier, strong acidic cation exchanger or polymers having acidic groups as the solid acid catalyst, and using the other reaction components, amounts and reaction conditions described above, including all the preferred embodiments.

In another preferred embodiment of the present invention 3-methylpent-1-en-4-yn-3-ol is isomerized to a mixture of Z-3-methylpent-2-en-4-yn-1-ol and E-3-methylpent-2-en-4-yn-1-ol employing diisopropylether as is/their solvent, Brønsted acids on a carrier, strong acidic cation exchanger or polymers having acidic groups as the solid acid catalyst, whereby the catalyst has a pore diameter of 100 to 500 Ǻ and a surface are of 20 to 500 m²/g (preferably 25 to 300 m²/g, more preferably 30 to 300 m²/g), and using the other reaction components, amounts and reaction conditions described above, including all the preferred embodiments.

In another more preferred embodiment of the present invention 3-methylpent-1-en-4-yn-3-ol is isomerized to a mixture of Z-3-methylpent-2-en-4-yn-1-ol and E-3-methylpent-2-en-4-yn-1-ol employing polymer bounded sulfonic acid as the solid acid catalyst and using the other reaction components, amounts and reaction conditions described above, including all the preferred embodiments.

In an even more preferred embodiment of the present invention 3-methylpent-1-en-4-yn-3-ol is isomerized to a mixture of Z-3-methylpent-2-en-4-yn-1-ol and E-3-methylpent-2-en-4-yn-1-ol employing diisopropylether as is/their solvent, polymer bounded sulfonic acid as the solid acid catalyst and using the other reaction components, amounts and reaction conditions described above, including all the preferred embodiments.

In a further preferred embodiment of the present invention 3-methylpent-1-en-4-yn-3-ol is isomerized to a mixture of Z-3-methylpent-2-en-4-yn-1-ol and E-3-methylpent-2-en-4-yn-1-ol employing diisopropylether as is/their solvent, Amberlyst or Dowex type polymers as the solid acid catalyst and using the other reaction components, amounts and reaction conditions described above, including all the preferred embodiments.

The reaction mixture obtained from the isomerization step comprises an aqueous phase and an organic solvent phase. In the further work-up a phase separation step followed by an optionally neutralization, e.g. with sodium carbonate, and separation of the organic solvent and not reacted tertiary allyl alcohol, e.g. pent-1-en-3-ol, is known in the art. Typically, the lower boiling components (e.g. organic solvent) and higher boiling components will be separated from the isomer mixture in one or more, preferably at least two devolatilization and/or distillation steps. Typically, the Z-pent-2-en-1-ol is then separated from the E-pent-2-en-1-ol by one or more final rectification steps.

The present invention is a process for isomerizing a pent-1-en-3-ol according to formula (1) 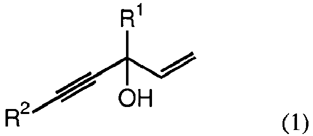 to a mixture of the stereoisomers Z-pent-2-en-1-ol (2) and E-pent-2-en-1-ol (3) according to formulae (2) and (3) 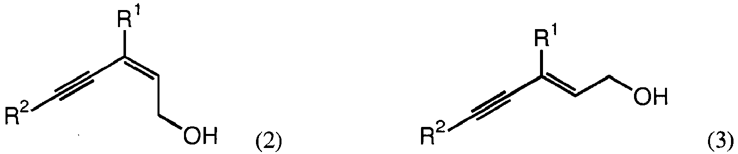 wherein
R¹ is selected from alkyl*, aryl (such as phenyl, naphthyl and tolyl), and alkyl*aryl (such as benzyl) radicals, preferably R¹ is selected from C₁ to C₁₅ alkyl* radicals (more preferably methyl radicals) and a phenyl radical,
R² is selected from H, alkyl*, and aryl (such as phenyl, naphthyl and tolyl) radicals, preferably R² is selected from H, C₁ to C₁₅ alkyl* radicals (more preferably C₁ to C₅ alkyl* radicals) and a phenyl radical;
more preferably R¹ is a methyl radical and R² is H;
which process comprises reacting the pent-1-en-3-ol (1) in a solvent and in the presence of a heterogeneous acid catalyst, wherein the solvent is a multiphase solvent system, wherein the multiphase solvent system comprises an aqueous phase and an organic solvent phase, and wherein the organic solvent phase comprises a water-immiscible organic solvent and wherein the water-immiscible organic solvent is diisopropyl ether.

* The alkyl radicals may be linear, branched or cyclic, preferably they are linear or branched, more preferably they are linear.

The present invention is carried out in a multiphase system, said multiphase system comprises an aqueous phase and an organic solvent phase. Thus, the aqueous phase and the organic solvent phase form 2 separate phases. Such a two-phase liquid solvent system is preferred. In the latter case the reaction mixture is a three-phase system: aqueous phase, organic solvent phase and catalyst.

It is especially advantageous when the water content in the reaction mixture is at least 5 weight-%.

Usually when using a multiphase (solvent) system problems with mass transport limitation occur. Surprisingly it was recognized that such problems do not occur in the multiphase solvent systems according to the present invention.

In a multiphase system, the reaction mixture obtained from the isomerization step comprises an aqueous phase and an organic solvent phase. In the further work-up a phase separation step followed by an optionally neutralization, e.g. with sodium carbonate, and separation of the organic solvent and not reacted tertiary allyl alcohol, e.g. pent-1-en-3-ol, is known in the art. Typically, the lower boiling components (e.g. organic solvent) and higher boiling components will be separated from the isomer mixture in one or more, preferably at least two devolatilization and/or distillation steps. Typically, the Z-pent-2-en-1-ol is then separated from the E-pent-2-en-1-ol by one or more final rectification steps.

Further advantages of this embodiment of the present invention are the long life time of the catalyst and that the process may also be performed in a continuous way.

The present invention is also directed to a process of preparing an isoprenoid selected from vitamin A, vitamin A derivatives, and carotenoids, which process comprises isomerizing a pent-1-en-3-ol according to the special embodiment of the present invention, isolating the Z-pent-2-en-1-ol and E-pent-2-en-1-ol, and converting the Z-pent-2-en-1-ol or E-pent-2-en-1-ol to the corresponding isoprenoid.

The present invention further relates to a process of preparing an isoprenoid selected from vitamin A, vitamin A derivatives, and carotenoids, which process comprises isomerizing 3-methylpent-1-en-4-yn-3-ol according to the special embodiment of the present invention, isolating the Z-3-methylpent-2-en-4-yn-1-ol and E-3-methylpent-2-en-4-yn-1-ol, and converting the Z-3-methylpent-2-en-4-yn-1-ol or E-3-methylpent-2-en-4-yn-1-ol to the corresponding isoprenoid.

When R¹ is a methyl radical and R² is H, which is most preferred, 3-methylpent-1-en-4-yn-3-ol according to formula (4) 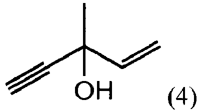 is isomerized to a mixture of the stereoisomers Z-3-methylpent-2-en-4-yn-1-ol according to formula (5) and E-3-methylpent-2-en-4-yn-1-ol according to formula (6)

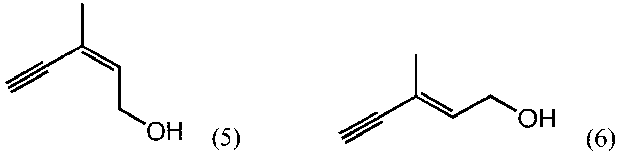

Preferably, the heterogeneous acid catalyst is selected from the group consisting of Brønsted acids on a carrier, strong acidic cation exchangers and polymers having acidic groups, i.e. functionalized polymers. Preferably the catalyst has a pore diameter in the range of from 100 to 500 Ǻ and/or a surface area of at least 20 m²/g (measured by nitrogen BET), preferably a surface area in the range of from 20 to 500 m²/g (more preferably in the range of from 25 to 300 m²/g, in the range of from 28 to 300 m²/g, in the range of from 28 to 70 m²/g, in the range of from 30 to 55 m²/g), **and/or** a total pore volume of at least 0.10 ml/g, preferably a total pore volume in the range of from 0.10 to 0.50 ml/g, more preferably a total pore volume in the range of from 0.15 to 0.45 ml/g, even more preferably a total pore volume in the range of from 0.18 to 0.42 ml/g, most preferably a total pore volume in the range of from 0.20 to 0.40 ml/g.

Even more preferably the acidic group of the catalyst is represented by sulphonic acid groups (-SO₃H) or by any other group having a pKₐ ≤ 4, preferably the acidic function of the catalyst is represented by sulphonic acid groups.

Thus, the catalyst may be an organic sulphonic acid on a carrier or a polymeric resin with functional sulphonic acid groups, whereby the polymeric resins with functional sulphonic acid groups are more preferred. The polymeric resins with functional sulphonic acid groups are also named "polymer bonded sulfonic acid". In a preferred embodiment a styrene-divinylbenzene polymer is used as matrix for the sulphonic acid groups.

A further suitable catalyst is a polymer functionalized with CO₂H groups.

The term "functionalized polymer with SO₃H groups" also encompasses polymers, where the SO₃H groups are bound to the polymer backbone via a linker. This linker may be an alkylene (such as e.g. propylene: -(CH₂)₃-), an arylene (such as p-tolylene) or an alkylarylene (such as e.g. benzylene: -CH₂-(C₆H₄)-). The same applies accordingly for the "functionalized polymer with CO₂H groups".

Of all the functionalized polymers cited the macroreticular polymers are preferred.

Preferably, the immobilized/solid Brønsted acid for use in the present invention has a pka value ≤ 4, more preferably ≤ 2, even more preferably ≤ 1.5 and most preferably ≤ 1.

Examples of Brønsted acids are organic sulphonic acids, H₂SO₄ and H₃PO₄.

The carrier may be either organic or inorganic. Examples of organic carriers are polymers. Examples of inorganic carriers are oxide carriers such as e.g. SiO₂, Al₂O₃, TiO₂, ZrO₂ and GeO₂.

Examples of Bronsted acids on a carrier are p-toluene sulphonic acid on a polymer (especially on a macroreticular polystyrene copolymer such as a macroreticular polystyrene divinylbenzene copolymer) or propyl sulphonic acid on silica.

Examples of suitable catalysts for the special embodiment of the present invention are Amberlyst type polymers, Lewatit, Dowex, und polymer bounded p-TsOH (p-toluene sulphonic acid), as well as Deloxan.

Most preferred solid acid catalysts are polymer-bounded sulphonic acids, e.g. Amberlyst-type (preferred) and Dowex-type polymers, i.e. bead form, macroreticular, strongly acidic ion exchange resins functionalized with sulphonic acid groups.

Of these the catalysts Amberlyst 15, 16 and 36, as well as Dowex DR-2030 are especially preferred, as well as the same catalysts sold under other tradenames. These catalysts are all functionalized styrene divinylbenzene copolymers.

In this embodiment furthermore this bead form, macroreticular, strongly acidic ion exchange resin functionalized with sulphonic acid groups has more preferably a concentration of the acid sites (i.e. the sulphonic acid groups) ≥ 4.0 eq/kg, preferably ≥ 4.5 eq/kg, even more preferably in the range of from 4.5 to 6.0 eq/kg, most preferably in the range of from 4.5 to 5.5 eq/kg. Additionally or alternatively this bead form, macroreticular, strongly acidic ion exchange resin functionalized with sulphonic acid groups has a surface area of at least 20 m²/g (measured by nitrogen BET), preferably a surface area in the range of from 20 to 70 m²/g, more preferably a surface area in the range of from 28 to 55 m²/g, **and/or** a total pore volume of at least 0.10 ml/g, preferably a total pore volume in the range of from 0.10 to 0.50 ml/g, more preferably a total pore volume in the range of from 0.15 to 0.45 ml/g, even more preferably a total pore volume in the range of from 0.18 to 0.42 ml/g, most preferably a total pore volume in the range of from 0.20 to 0.40 ml/g.

More preferably the bead form, macroreticular, strongly acidic ion exchange resin functionalized with sulphonic acid groups has a concentration of the acid sites as given above, a surface area as given above **and** a total pore volume as given above.

Thus, the most preferred heterogeneous acid catalysts of the present invention are bead form, macroreticular, strongly acidic ion exchange resins functionalized with sulphonic acid groups having a concentration of the acid sites (i.e. the sulphonic acid groups) ≥ 4.0 eq/kg (preferably ≥ 4.5 eq/kg, more even more preferably in the range of from 4.5 to 6.0 eq/kg, most preferably in the range of from 4.5 to 5.5 eq/kg), a surface area of at least 20 m²/g (measured by nitrogen BET) (preferably in the range of from 20 to 70 m²/g, more preferably in the range of from 28 to 55 m²/g), an average pore diameter of at least 200 Å (preferably in the range of from 200 to 350 A) **and** a total pore volume of at least 0.10 ml/g (preferably in the range of from 0.10 to 0.50 ml/g, more preferably a total pore volume in the range of from 0.15 to 0.45 ml/g, even more preferably a total pore volume in the range of from 0.18 to 0.42 ml/g, most preferably a total pore volume in the range of from 0.20 to 0.40 ml/g).

Furthermore polymers with SO₃H groups as prepared according to the processes of EP-A 507 490 and US 5,081,160, whose contents are incorporated herein by reference, may be also used as catalyst in the isomerization reaction according to this embodiment.

Although it is not mandatory, a stabilizer, such as hydroquinone or 2,6-di-tert-butyl-p-cresol or a mixture thereof, may be added to the reaction mixture. If used, the stabilizer, e.g. hydroquinone or 2,6-di-tert-butyl-p-cresol or a mixture thereof, is typically added in an amount of from 1 to 10,000 ppm by weight, preferably from 5 to 1,000 ppm by weight, more preferably from 7 to 500 ppm by weight, and most preferably from 10 to 100 ppm by weight, each based on the total weight of the multiphase system.

Further stabilizers may be other anti-oxidants.

Typically, the solid acid catalyst is mixed with water and added to the process, whereby preferably so much water is added so that the water content in the reaction mixture is at least 5 weight-%.

Compared to the prior art methods the separation and recycling of the catalyst is much more convenient also in this embodiment. In a multiphase system, the solid catalyst and the aqueous phase of the multiphase system may easily be separated from the product layer and recycled. That means that the catalyst may be recycled. It has been found that the catalyst gives still high yield and conversion even if used several times.

Usually, the pent-1-en-3-ol is soluble in the organic solvent used (diisopropyl ether) and thus, it is added to the process as a solution in the organic solvent. In one embodiment, the concentration of the pent-1-en-3-ol in the organic solvent ranges from 5 to 80% by weight, preferably from 15 to 60% by weight, more preferably from 20 to 50% by weight, and most preferably from 25 to 45% by weight.

The pent-1-en-3-ol may, however, be also added in the reaction chamber/reactor as such. Preferably the pent-1-en-3-ol is added as a solution in the organic solvent.

The various components may be added in any order. For example, the aqueous mixture of the solid acid catalyst may be loaded into the reaction vessel and then the solution of the pent-1-en-3-ol in the organic solvent may be added or vice versa. It is also possible to divide the total amount of a component to be added and add the partial amounts at various time points in the process.

If the reaction is carried out discontinuously the mixing of the reaction components is done by agitation of the reaction mixture, e.g. by stirring. Preferably, the isomerization reaction is conducted in a stirred tank reactor.

If the reaction is carried out in continuous manner a plug-flow reactor with a fixed-bed is used, or a continuous stirred tank reactor. A cascade reactor system may also be used.

The isomerization reaction is typically conducted at a temperature in the range of from 20 to 80°C, depending on the type of the reaction components used and the other reaction conditions applied. At atmospheric pressure the reaction temperature is preferably in the range of from 30 to 70°C, more preferably in the range of from 35 to 65°C, and most preferably in the range of from 40 to 60°C.

The isomerization reaction has, depending on the used set-up and conditions, reaction times in the range of from 1 to 25 h. At a typical temperature range the reaction time is preferably in the range of from 1.5 to 8 h, more preferably in the range of from 2 to 6 h, and most preferably in the range of from 2 to 5 h.

Typically, this embodiment is also conducted at a pent-1-en-3-ol conversion of at least 70%, preferably at least 81%, more preferably at least 86%, even more preferably at least 89%, and most preferably at least 92%.

### Further objects of the present invention

The present invention also relates to a process of preparing an isoprenoid selected from vitamin A, vitamin A derivatives, and carotenoids, which process comprises isomerizing a pent-1-en-3-ol according to the present invention as disclosed above, isolating the Z-pent-2-en-1-ol and E-pent-2-en-1-ol, and converting the Z-pent-2-en-1-ol or E-pent-2-en-1-ol to the corresponding isoprenoid.

Z-3-methylpent-2-en-4-yn-1-ol obtained in major amounts by a preferred embodiment of the present isomerization reaction is an intermediate product for the syntheses of vitamin A or vitamin A derivatives. One of the most economically successful processes for the preparation of vitamin A and its derivatives is the Isler synthesis of 1948. The conversion of Z-3-methylpent-2-en-4-yn-1-ol to vitamin A or a vitamin A derivative is for example described in US-A-2,451,739 to Otto Isler. E-3-methylpent-2-en-4-yn-1-ol obtained in minor amounts by the preferred embodiment of the present isomerization reaction also is a useful intermediate. It may be used to synthesize astaxanthin, zeaxanthin and further carotenoids. An illustrative synthesis of astaxanthin is described in EP-A-0 005 748. A different synthesis of astaxanthin also using E-3-methylpent-2-en-4-yn-1-ol as starting C₆ component is taught in CN-A-166 803.

The invention will now be further illustrated in the following non-limiting examples.

### EXAMPLES

In the following examples the terms "yield" and "selectivity" both refer to the sum of the desired products, i.e. the sum of the Z- and E-isomers.

The reactions were carried out in a flask equipped with a stirrer (mechanical or magnetical), a thermometer and a reflux condenser with Ar overlay. This will be heated with an oil bath filled with WACKER Silicon oil AP 100.

### Chemicals

Pure 3-methylpent-1-en-4-yn-3-ol (97%), as well as 46, 48 and 50 weight-% solutions of 3-methylpent-1-en-4-yn-3-ol in iPr₂O (diisopropylether) were obtained from DSM Nutritional Products Ltd, Sisseln, Switzeland. Diisopropylether (99%) itself was also obtained therefrom. It was stabilized with 2,6-di-ter-butyl-p-cresol (99%), which was purchased from Fluka.

The catalysts Amberlyst® 15, Amberlyst® 16 and Amberlyst® 36 were purchased from Rohm & Haas, (Springhouse, Philadelphia, USA).

The catalyst Dowex DR-2030 was purchased from Fluka.

### Example 1: Procedure for batch experiments in water/diisopropyl ether

In the reactor, 20 g dry solid acid catalyst (Amberlyst 36 Dry) was placed. Under stirring at 350 rpm at 294 K 100 ml of water was added. A mixture of 100 ml 50 weight-% 3-methylpent-1-en-4-yn-3-ol (0.42 mol) in diiso-propyl ether was added. The reaction mixture was heated up to 328 K and stirred for 5 h. After cooling to room temperature the solid phase was separated from the liquid phases and washed two times with 30 ml diisopropyl ether. The water phase was separated from the organic layer and replaced in the reaction flask with the catalyst. The organic layer was washed two times with 10 ml water and concentrated at 200 mbar, 35°C in 30 min. The crude product (60 g) was analyzed by GC with internal standard. E- and Z-3-methylpent-2-en-4-yn-1-ol could be synthesized at 98% conversion in 90% yield.

### Example 2

Example 1 was repeated with 40 g of Amberlyst 36 Wet and 80 ml water. The result was the same as in example 1.

### Example 3: General procedure for the continuous process

The plug-flow reactor was filled with the catalyst (Amberlyst 36) and the 3-methylpent-1-en-4-yn-3-ol in a multiphase solvent, e.g a diisopropyl ether water mixture, was pumped through the static mixer and the reactor. After the reaction mixture has passed the reactor it was collected and analyzed. The reactor jacket was heated to the desired reaction temperature. Samples were taken for GC-analysis to follow the progression of the reaction. The catalyst life-time was > 150 hours without loss on activity and selectivity.

### Example 4: Procedure for batch experiments in water/diisopropyl ether

In the reactor, 20 g dry solid acid catalyst (Dowex DR-2030) was placed. Under stirring at 350 rpm at 294 K 100 ml of water was added. A mixture of 100 ml 50 weight-% 3-methylpent-1-en-4-yn-3-ol (0.42 mol) in diisopropyl ether was added. The reaction mixture was heated up to 328 K and stirred for 5 h. After cooling to room temperature the solid phase was separated from the liquid phases and washed two times with 30 ml diisopropyl ether. The water phase was separated from the organic layer and replaced in the reaction flask with the catalyst. The organic layer was washed two times with 10 ml water and concentrated at 200 mbar, 35°C in 30 min. The crude product (60 g) was analyzed by GC with internal standard. E- and Z-3-methylpent-2-en-4-yn-1-ol could be synthesized at 95% conversion in 86% yield.

### Example 5: Recycling of catalyst

In a series of experiments with the experimental set-up as described in examples 1 and 4, the catalyst (Amberlyst 36, Amberlyst 15, Amberlyst 16, Dowex DR-2030) were re-used after separation of the reaction mixture. The catalysts could be re-used in minimum 5 times without decrease in selectivity and yield.

| **Catalyst** | **conversion (after 5^{th} run) in %** | **yield (after 5^{th} run) in %** |
|---|---|---|
| Amberlyst 36 | 96 | 90 |
| Amberlyst 15 | 98 | 83 |
| Amberlyst 16 | 96 | 86 |
| Dowex DR20-30 | 95 | 84 |

## Claims

1. A process for isomerizing a pent-1-en-3-ol according to formula (1) 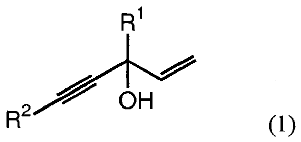 to a mixture of the stereoisomers Z-pent-2-en-1-ol (2) and E-pent-2-en-1-ol (3) according to formulae (2) and (3) 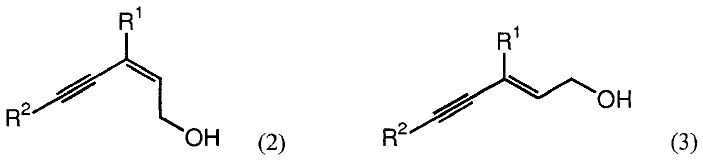 wherein
R¹ is selected from alkyl, aryl, and alkylaryl radicals,
R² is selected from H, alkyl, and aryl radicals;
which process comprises reacting the pent-1-en-3-ol (1) in a solvent and in the presence of a heterogeneous acid catalyst, wherein the solvent is a multiphase solvent system, wherein the multiphase solvent system comprises an aqueous phase and an organic solvent phase, and wherein the organic solvent phase comprises a water-immiscible organic solvent and wherein the water-immiscible organic solvent is diisopropyl ether..

2. The process according to claim 1 wherein R¹ is selected from C₁ to C₁₅ alkyl radicals and a phenyl radical, R² is selected from H, C₁ to C₁₅ alkyl radicals and a phenyl radical.

3. The process according to any one ore more of the preceding claims wherein the reaction mixture is a three-phase system: aqueous phase, organic solvent phase and catalyst.

4. The process according to any one of the preceding claims wherein the heterogeneous acid catalyst is selected from the group consisting of Brønsted acids on a carrier, strong acidic cation exchangers and polymers having acidic groups.

5. The process according to any of claims 1-4 wherein the heterogeneous acid catalyst has a pKa-value ≤ 4.

6. The process according to any of claims 1-4 wherein the heterogeneous acid catalyst has a pKa-value ≤ 2.

7. The process according to any of claims 1-4 wherein the heterogeneous acid catalyst has a pKa-value ≤ 1.5.

8. The process according to any of claims 1 to 7, wherein the heterogeneous acid catalyst is a bead form, macroreticular, sulphonic acid, strongly acidic ion exchange resin.

9. The process according to claim 8, wherein the bead form, macroreticular, sulphonic acid, strongly acidic ion exchange resin has a surface area of at least 20 m²/g (measured by nitrogen BET), **and/or** a total pore volume of at least 0.10 ml/g.

10. The process according to claim 8, wherein the bead form, macroreticular, sulphonic acid, strongly acidic ion exchange resin has a surface area in the range of from 20 to 70 m²/g.

11. The process according to claim 9 or 10, wherein the bead form, macroreticular, sulphonic acid, strongly acidic ion exchange resin has a total pore volume in the range of from 0.10 to 0.50 ml/g.

12. The process according to any one of the preceding claims **characterized in that** the conversion of isomerizing pent-1-en-3-ol according to formula (1) to the mixture of the stereoisomers Z-pent-2-en-1-ol (2) and E-pent-2-en-1-ol (3) is at least 70%.

13. The process according to any one of the preceding claims **characterized in that** the conversion of isomerizing pent-1-en-3-ol according to formula (1) to the mixture of the stereoisomers Z-pent-2-en-1-ol (2) and E-pent-2-en-1-ol (3) is at least 81%.

14. A process of preparing an isoprenoid selected from vitamin A, vitamin A derivatives, and carotenoids, which process comprises isomerizing a pent-1-en-3-ol according to any of the preceding claims, isolating the Z-pent-2-en-1-ol and E-pent-2-en-1-ol, and converting the Z-pent-2-en-1-ol or E-pent-2-en-1-ol to the corresponding isoprenoid.

15. The process according to claim 14 wherein the carotenoids are astaxanthin and zeaxanthin.

## Patentansprüche

1. Verfahren zur Isomerisierung eines Pent-1-en-3-ols gemäß der Formel (1) 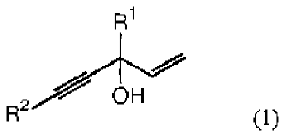 zu einem Gemisch der Stereoisomeren Z-Pent-2-en-1-ol (2) und E-Pent-2-en-1-ol (3) gemäß den Formeln (2) und (3) 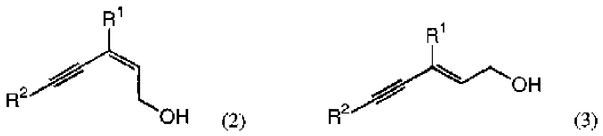 wobei
R¹ aus Alkyl-, Aryl- und Alkylarylresten ausgewählt ist,
R² aus H, Alkyl- und Arylresten ausgewählt ist; wobei das Verfahren die Umsetzung des Pent-1-en-3-ols (1) in einem Lösungsmittel und in Gegenwart eines heterogenen sauren Katalysators umfasst, wobei es sich bei dem Lösungsmittel um ein mehrphasiges Lösungsmittelsystem handelt, wobei das mehrphasige Lösungsmittelsystem eine wässrige Phase und eine organische Lösungsmittelphase umfasst und wobei die organische Lösungsmittel-phase ein mit Wasser nicht mischbares organisches Lösungsmittel umfasst und wobei es sich bei dem mit Wasser nicht mischbaren organischen Lösungsmittel um Diisopropylether handelt.

2. Verfahren nach Anspruch 1, wobei R¹ aus C₁- bis C₁₅-Alkylresten und einem Phenylrest, R² aus H, C₁- bis C₁₅-Alkylresten und einem Phenylrest ausgewählt ist.

3. Verfahren nach einem oder mehreren der vorhergehenden Ansprüche, wobei es sich bei dem Reaktionsansatz um ein Drei-Phasen-System handelt: wässrige Phase, organische Lösungsmittelphase und Katalysator.

4. Verfahren nach einem der vorhergehenden Ansprüche, wobei der heterogene saure Katalysator aus der aus Brønsted-Säuren an einem Träger, stark sauren Kationenaustauschern und Polymeren mit sauren Gruppen bestehenden Gruppe ausgewählt ist.

5. Verfahren nach einem der Ansprüche 1-4, wobei der heterogene saure Katalysator einen pKa-Wert ≤ 4 aufweist.

6. Verfahren nach einem der Ansprüche 1-4, wobei der heterogene saure Katalysator einen pKa-Wert ≤ 2 aufweist.

7. Verfahren nach einem der Ansprüche 1-4, wobei der heterogene saure Katalysator einen pKa-Wert ≤ 1,5 aufweist.

8. Verfahren nach einem der Ansprüche 1 bis 7, wobei es sich bei dem heterogenen sauren Katalysator um ein makroretikuläres, stark saures Sulfonsäure-Ionenaustauschharz in Kügelchenform handelt.

9. Verfahren nach Anspruch 8, wobei das makroretikuläre, stark saure Sulfonsäure-Ionenaustauschharz in Kügelchenform eine Oberfläche von wenigstens 20 m²/g (gemessen mit Stickstoff-BET) und/oder ein Gesamtporenvolumen von wenigstens 0,10 ml/g aufweist.

10. Verfahren nach Anspruch 8, wobei das makroretikuläre, stark saure Sulfonsäure-Ionenaustauschharz in Kügelchenform eine Oberfläche im Bereich von 20 bis 70 m²/g aufweist.

11. Verfahren nach Anspruch 9 oder 10, wobei das makroretikuläre, stark saure Sulfonsäure-Ionenaustauschharz in Kügelchenform ein Gesamtporenvolumen im Bereich von 0,10 bis 0,50 ml/g aufweist.

12. Verfahren nach einem der vorhergehenden Ansprüche, **dadurch gekennzeichnet, dass** der Umsatz der Isomerisierung von Pent-1-en-3-ol gemäß der Formel (1) zum Gemisch der Stereoisomeren Z-Pent-2-en-1-ol (2) und E-Pent-2-en-1-ol (3) bei wenigstens 70% liegt.

13. Verfahren nach einem der vorhergehenden Ansprüche, **dadurch gekennzeichnet, dass** der Umsatz der Isomerisierung von Pent-1-en-3-ol gemäß der Formel (1) zum Gemisch der Stereoisomeren Z-Pent-2-en-1-ol (2) und E-Pent-2-en-1-ol (3) bei wenigstens 81% liegt.

14. Verfahren zur Herstellung eines aus Vitamin A, Vitamin-A-Derivaten und Carotinoiden ausgewählten Isoprenoids, bei dem man ein Pent-1-en-3-ol nach einem der vorhergehenden Ansprüche isomerisiert, das Z-Pent-2-en-1-ol und E-Pent-2-en-1-ol isoliert und das Z-Pent-2-en-1-ol oder E-Pent-2-en-1-ol in das entsprechende Isoprenoid überführt.

15. Verfahren nach Anspruch 14, wobei es sich bei den Carotinoiden um Astaxanthin und Zeaxanthin handelt.

## Revendications

1. Procédé pour l'isomérisation d'un pent-1-én-3-ol selon la formule (1) 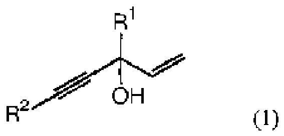 en un mélange des stéréoisomères Z-pent-2-én-1-ol (2) et E-pent-2-én-1-ol (3) selon les formules (2) et (3) 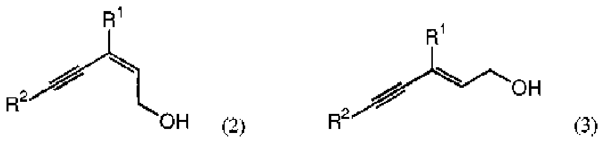 dans lesquelles
R¹ est choisi parmi des radicaux alkyle, aryle et alkylaryle,
R² est choisi parmi H, des radicaux alkyle et aryle ; Lequel procédé comprend la mise en réaction du pent-1-én-3-ol (1) dans un solvant et en la présence d'un catalyseur acide hétérogène, le solvant étant un système de solvant multiphasique, le système de solvant multiphasique comprenant un phase aqueuse et une phase de solvant organique, et la phase de solvant organique comprenant un solvant organique non miscible à l'eau et le solvant organique non miscible à l'eau étant l'éther diisopropylique.

2. Procédé selon la revendication 1, dan lequel R est choisi parmi des radicaux alkyle en C₁-C₁₅ et un radical phényle, R² est choisi parmi H, des radicaux alkyle en C₁-C₁₅ et un radical phényle.

3. Procédé selon une ou plusieurs des revendications précédentes, dans lequel le mélange réactionnel est un système triphasique : phase aqueuse, phase de solvant organique et catalyseur.

4. Procédé selon l'une quelconque des revendications précédentes, dans lequel le catalyseur acide hétérogène est choisi dans le groupe constitué par des acides de Brønsted sur un support, des échangeurs de cations fortement acides et des polymères comportant des groupes acides.

5. Procédé selon l'une quelconque des revendications 1 à 4, dans lequel le catalyseur acide hétérogène a un pKa ≤ 4.

6. Procédé selon l'une quelconque des revendications 1 à 4, dans lequel le catalyseur acide hétérogène a un pKa ≤ 2.

7. Procédé selon l'une quelconque des revendications 1 à 4, dans lequel le catalyseur acide hétérogène a un pKa ≤ 1,5.

8. Procédé selon l'une quelconque des revendications 1 à 7, dans lequel le catalyseur acide hétérogène est une résine échangeuse d'ions fortement acide, de type acide sulfonique, macroréticulaire, sous forme de billes.

9. Procédé selon la revendication 8, dans lequel la résine échangeuse d'ions fortement acide, de type acide sulfonique, macroréticulaire, sous forme de billes, a une superficie d'au moins 20 m²/g (mesurée par la méthode BET à l'azote), et/ou un volume total de pores d'au moins 0,10 ml/g.

10. Procédé selon la revendication 8, dans lequel la résine échangeuse d'ions fortement acide, de type acide sulfonique, macroréticulaire, sous forme de billes, a une superficie dans la plage de 20 à 70 m²/g.

11. Procédé selon la revendication 9 ou 10, dans lequel la résine échangeuse d'ions fortement acide, de type acide sulfonique, macroréticulaire, sous forme de billes, a un volume total de pores dans la plage de 0,10 à 0,50 ml/g.

12. Procédé selon l'une quelconque des revendications précédentes, **caractérisé en ce que** la conversion de l'isomérisation du pent-1-én-3-ol selon la formule (1) en le mélange des stéréoisomères Z-pent-2-én-1-ol (2) et E-pent-2-én-1-ol (3) est d'au moins 70 %.

13. Procédé selon l'une quelconque des revendications précédentes, **caractérisé en ce que** la conversion de l'isomérisation du pent-1-én-3-ol selon la formule (1) en le mélange des stéréoisomères Z-pent-2-én-1-ol (2) et E-pent-2-én-1-ol (3) est d'au moins 81 %.

14. Procédé pour la préparation d'un isoprénoïde choisi parmi la vitamine A, des dérivés de vitamine A et des caroténoïdes, lequel procédé comprend l'isomérisation d'un pent-1-én-3-ol selon l'une quelconque des revendications précédentes, l'isolement du Z-pent-2-én-1-ol et du E-pent-2-én-1-ol et la conversion du Z-pent-2-én-1-ol ou du E-pent-2-én-1-ol en l'isoprénoïde correspondant.

15. Procédé selon la revendication 14, dans lequel les caroténoïdes sont l'astaxanthine et la zéaxanthine.
